# EUROPEAN PATENT APPLICATION

(11) **EP 1 690 518 A1**
(43) Date of publication of application: **16.08.2006**
(21) Application number: 04799456.1
(22) Date of filing: 02.11.2004
(51) Int. Cl.: A61J 3/07, A23P 1/04, B01J 2/06

(54) **SEAMLESS CAPSULE MANUFACTURING METHOD, SEAMLESS CAPSULE MANUFACTURING DEVICE, AND SEAMLESS CAPSULE**

(30) Priority: 07.11.2003 JP 2003378625
(71) Applicant: Freund Corporation, Tokyo 163-6034 (JP)
(72) Inventor: YAMANAKA, Kuniaki c/o Freund Corporation, Shinjuku-ku, Tokyo 1636034 (JP); UNOSAWA, Kazuomi c/o Freund Corporation, Shinjuku-ku, Tokyo 1636034 (JP); TAKEI, Narimichi c/o Freund Corporation, Shinjuku-ku, Tokyo 1636034 (JP); KONNO, Shouji c/o Freund Corporation, Shinjuku-ku, Tokyo 1636034 (JP); IKEDA, Masayuki c/o Freund Corporation, Shinjuku-ku, Tokyo 1636034 (JP)
(74) Representative: HOFFMANN EITLE
(86) International application number: PCT/JP2004/016250
(87) International publication number: WO 2005/044174

(57) **Abstract**

A seamless capsule manufacturing device comprises a multiple nozzle 7 for ejecting liquid drops into hardening liquid 10 and a flow passage tube 11 for containing hardening liquid 10. The flow passage tube 11 has a deformation section 28 that includes an inlet part 25 and a formation tube part 28b showing a cross sectional area smaller than the inlet part 25. The liquid drops ejected from the multiple nozzle 7 into the hardening liquid 10 are once made to become spherical liquid drops 26 in a sol state at the inlet part 25. The liquid drops 26b are introduced from the inlet part 25 into the deformation section 28 while they are still in a sol state. As hardening liquid 10 is introduced from the inlet part 25 into the formation tube part 28b, its flow rate is changed and the liquid drops 26 are deformed due to the change in the flow rate to produce nonspherical seamless capsules SC. With this arrangement, it is possible to provide a method and a device for manufacturing high quality nonspherical seamless capsules with an enhanced level of productivity without requiring cumbersome parameter defining operations and delicate control operations.

## Description

### Technical Field

This invention relates to the technology of manufacturing seamless capsules by coating drops of a filling substance such as food, dietary food, medicine, aromatic or spice with a film containing gelatin or agar. More specifically, the invention relates to a method of manufacturing nonspherical seamless capsules.

### Background Art

Conventionally, many seamless capsules for use in the field of medicines and so on are manufactured by means of a so-called dropping method. A multiple nozzle is used for the dropping method. Thus, in the case of manufacturing two-layered capsules, a double nozzle having an ejection port for ejecting the capsule filling substance and an ejection port for ejecting the film forming substance respectively arranged at the inside and at the outside is used. The filling substance and the film forming substance are ejected from the tips of the respective nozzles into hardening liquid and the ejected liquid drops take a spherical form due to the surface tension thereof. Then, the liquid drops are cooled and hardened in the hardening liquid that is forced to circulate at a constant speed to make spherical seamless capsules.

There has been an increasing demand for nonspherical capsules such as ellipsoidal capsules and oblong capsules in recent years for the purpose of easy ingestion, easy handling and differentiation of marketing. However, the above-described dropping method can only be used for manufacturing spherical capsules because it is a manufacturing method of utilizing surface tension and therefore ellipsoidal capsules have been and being manufactured exclusively by means of an ordinary sheet method.

Various techniques have been proposed for forming capsules having a rugby ball profile or an ellipsoidal profile by means of the dropping method in an attempt to manufacture nonspherical seamless capsules. For example, Patent Document 1 in the list shown below discloses a method of manufacturing deformed soft seamless capsules by forcing liquid drops dropped from a nozzle to pass through a narrow tube having a diameter smaller than the diameter of the liquid drops in a state where the liquid drops are still in a sol state and are then cooled and hardened so as to take an ellipsoidal form. Patent Document 2 in the list shown below discloses a method of manufacturing deformed soft seamless capsules by molding/cooling liquid drops dropped from a nozzle in respective molds like a drawing die in a sol state. Patent Document 3 in the list shown below discloses a method of manufacturing ellipsoidal seamless micro capsules by producing a recess on each liquid drop at the front end of a nozzle so as to mold a capsule having a rugby ball profile from the liquid drop after dropping and hardening it. Finally, Patent Document 4 in the list shown below discloses a method of manufacturing nonspherical capsules by heating seamless capsules prepared in advance and held in a gel state into a sol state, forcing them to pass through a molding jig having a part of a diameter smaller than the diameter of the seamless capsules to deform them and show a nonspherical profile and subsequently cooling them into a gel state.
Patent Document 1: Japanese Patent Publication No. 60-46980
Patent Document 2: Japanese Patent Publication No. 60-46981
Patent Document 3: Japanese Patent Publication No. 61-17541
Patent Document 4: Japanese Patent Application Laid-Open
Publication No. 2000-325431

### Disclosure of the Invention

However, the above-cited known methods of manufacturing nonspherical capsules are accompanied by the following problems. Firstly, with the method of Patent Document 3, the cooling flow for forming a recess on each liquid drop requires a very delicate operation of adjusting the cooling flow for forming a recess on a liquid drop and it is very difficult- to regulate the cutting flow and the recess forming flow to an optimal condition. Additionally, the cooling flow has to be adjusted each time the capsule size is or the capsule ingredients are changed to entail a cumbersome adjusting operation.

Next, the methods of Patent Documents 1, 2 and 4 involve a problem that the narrow tube and the mold for forming deformed nonspherical capsules can easily become clogged with liquid drops. Once the tube and/or the mold is clogged, it is no longer possible to continue the manufacture of capsules. Then, the operation of the manufacturing device has to be suspended to remove the clogging capsule and/or the tube has to be replaced. Thus, the efficiency of manufacturing nonspherical capsules falls remarkably if a clogging capsule appears frequently and hence there is a demand for an improved nonspherical capsule manufacturing method.

Additionally, these methods are also accompanied by a problem that it is difficult to control the flow volume and the temperature of the liquid for hardening capsules. Generally, when capsules are manufactured by means of a dropping type seamless capsule manufacturing device, the quality of manufactured capsules (in terms of weight, precision, diameter, oil drop, degree of uneven wall thickness etc.) largely depends on the control of the flow volume (flow rate) of hardening liquid in the capsule manufacturing process. However, since liquid drops are forced to pass through a narrow tube or a mold, the flow of hardening liquid can often be pulsated and/or blocked by the resistance of liquid drops that arises when the latter are deformed. As a result, the quality of finished capsules is not stable and not only give rise to dispersion of weight and uneven wall thickness of capsules but also to a problem of a low productivity and a poor industrialization feasibility.

An object of the present invention to provide a method and a device for manufacturing nonspherical capsules that can produce high quality nonspherical seamless capsules with an enhanced level of productivity without requiring cumbersome setting operations and delicate control operations.

In the present invention, a seamless capsule manufacturing method of manufacturing seamless capsules by ejecting liquid drops from a nozzle into hardening liquid and hardening at least a surface part of each liquid drop, characterized in that each liquid drop is deformed to show a nonspherical profile by changing the flow rate of hardening liquid while the liquid drop is still in a sol state. In a seamless capsule manufacturing method according to the invention, the liquid drop may be drawn to expand in the direction of the flow path by increasing the flow rate of hardening liquid.

Thus, according to the present invention, the liquid drops that are ejected from a nozzle into hardening liquid come to show a spherical profile once in a sol state in the hardening liquid. Then, as the flow rate of hardening liquid is changed while the spherical liquid drops are still held in a sol state, the liquid drops are deformed as a function of the change in the flow rate and turned to nonspherical liquid drops. Neither a narrow tube nor a mold having a diameter smaller than the diameter of the ejected spherical liquid drops is used to deform the liquid drops by means of the manufacturing method according to the present invention and simply the flow rate of hardening liquid is changed in the molding process. Therefore, the tube or the like is prevented from being clogged and the flow of hardening liquid is prevented from being pulsated to consequently improve the quality of produced capsules and the productivity of manufacturing capsules.

Preferably, in a seamless capsule manufacturing method according to the invention, the formed nonspherical seamless capsules may be subjected to a contact process of being brought into contact with ethanol type processing liquid. With this arrangement, the change in the profile of seamless capsules, if any, is suppressed in a subsequent drying step so that the deformed nonspherical seamless capsules are made to maintain a good profile after the drying process.

In another aspect of the present invention, there is provided a seamless capsule manufacturing device comprising a nozzle for ejecting liquid for forming capsules and a flow passage tube containing hardening liquid for hardening at least a surface part of each liquid drop formed from the liquid, characterized in that the flow passage tube has an inlet part exposed to the nozzle so as to receive the liquid ejected/supplied from the nozzle and a deformation section having a cross sectional area smaller than the inlet part.

Thus, according to the present invention, the liquid drops that are ejected from the nozzle into hardening liquid come to show a spherical profile once in a sol state in the inlet part of the flow passage tube. Then, they are introduced into the deformation section from the inlet part while the spherical liquid drops are still held in a sol state. The deformation section has a cross sectional area smaller than the inlet part so that, as hardening liquid is introduced from the inlet part into the deformation section, the flow rate of hardening liquid changes. As the flow rate of hardening liquid changes, the liquid drops are deformed as a function of the change in the flow rate to produce nonspherical seamless capsules. Neither a narrow tube nor a mold having a diameter smaller than the diameter of the ejected liquid drops is used to deform the spherical liquid drops by means of a manufacturing device according to the present invention and simply the flow rate of hardening liquid in the deformation section is changed in the molding process. Therefore, the tube or the like is prevented from being clogged and the flow of hardening liquid is prevented from being pulsated to consequently improve the quality of produced capsules and the productivity of manufacturing capsules.

Preferably, in a seamless capsule manufacturing device according to the present invention, the deformation section is arranged downstream relative to the inlet part at a position where the ejected liquid drops arrive in a sol state. With this arrangement, nonspherical capsules can be formed with ease because the ejected spherical liquid drops are still in a sol state. Additionally, the cross section of the deformation section may be circular, elliptic, polygonal or of some other shape having one or more than one straight parts. For the purpose of the present invention, the deformation section may be immediately linked to the inlet part and the deformation section may be provided at the inlet part side thereof with a tapered introducing section.

Preferably, the inner diameter D₁ of the deformation section is greater than the diameter D₀ of the ejected liquid drops and not greater than three times of the diameter D₀ of the ejected liquid drops in the inlet part (D₀ < D₁ ≤ 3D₀) . The inner diameter D₁ may be between (1/6) times and (2/3) times, preferably between (1/5) times and (3/5) times, more preferably between (1/4) times and (1/2) times of the inner diameter D₂ of the flow passage tube in the inlet part. For the purpose of the present invention, the inner diameter D₁ of the deformation section is defined to be the diameter of the largest circle that can be inscribed in the cross section of the deformation section. The diameter D₀ of the ejected liquid drops in the inlet part is defined to be equal to the diameter of the volume obtained as quotient of division of dividing the flown volume of the capsule forming liquid ejected from the nozzle per unit time by the number of liquid drops produced per unit time, assuming that the volume takes a spherical shape.

Preferably, the cross sectional area S of the deformation section is within a range of (n/4)D₀² < S ≤ (9π/4) D₀², where D₀ is the diameter of the ejected liquid drops in the inlet part. The cross sectional area S is preferably between (1/36) times and (4/9) times, more preferably between (1/25) times and (4/25) times, most preferably between (1/16) times and (1/4) times of the cross sectional area of the flow passage tube in the inlet part. When the inner diameter D₁ and the cross sectional area S of the deformation section are defined as above, it is possible to achieve a preferable change in the flow rate of hardening liquid for transforming spherical liquid drops into nonspherical capsules. In other words, when the inner diameter and the cross sectional area are out of the above cited respective ranges, it is not possible to achieve a satisfactory change in the flow rate so that the liquid drops may be deformed insufficiently or may not be deformed at all if the change in the flow rate is not sufficient and some of the liquid drops may be ruptured or otherwise become unsatisfactory if the change in the flow rate is too large.

A seamless capsule manufacturing method according to the present invention is characterized in that nonspherical seamless capsules are manufactured by means of a seamless capsule manufacturing device according to the present invention. In a seamless capsule manufacturing method according to the present invention, the produced seamless capsules may be subjected to a contact process of being brought into contact with ethanol type treatment liquid. With this arrangement, any possible undesirable deformation of the produced capsules is suppressed in the drying step and the seamless capsules are held to a favorably deformed state after the drying step.

A nonspherical seamless capsule according to the present invention is characterized in that it is obtained by a seamless capsule manufacturing method according to the present invention.

With a seamless capsule manufacturing method according to the present invention, liquid drops are ejected from a nozzle into hardening liquid. Then, as the flow rate of hardening liquid is changed while the liquid drops are still held in a sol state, the ejected liquid drops are deformed as a function of the change in the flow rate and turned to nonspherical liquid drops. Neither a narrow tube nor a mold having a diameter smaller than the diameter of the ejected spherical liquid drops is used to deform the liquid drops and simply the flow rate of hardening liquid is changed in the molding process. Therefore, the tube or the like is prevented from being clogged and the flow of hardening liquid is prevented from being pulsated to consequently improve the quality of produced capsules and the productivity of manufacturing capsules.

Since a seamless capsule manufacturing device according to the present invention comprises a nozzle for ejecting liquid for forming capsules and a flow passage tube containing hardening liquid for hardening at least a surface part of each liquid drop formed from the liquid and the flow passage tube has an inlet part for receiving the liquid ejected/supplied from the nozzle and a deformation section having a cross sectional area smaller than the inlet part, the ejected liquid drops are deformed and turned into nonspherical seamless capsules by the change in the flow rate of hardening liquid due to the change in the cross sectional area. Neither a narrow tube nor a mold having a diameter smaller than the diameter of the ejected spherical liquid drops is used in the process of molding liquid drops. Therefore, the tube or the like is prevented from being clogged and the flow of hardening liquid is prevented from being pulsated to consequently improve the quality of produced capsules and the productivity of manufacturing capsules.

On the other hand, in a seamless capsule manufacturing method according to the invention, the formed nonspherical seamless capsules are preferably subjected to a contact process of being brought into contact with ethanol type processing liquid. With this arrangement, the change in the profile of seamless capsules, if any, is suppressed in a subsequent drying step so that the deformed nonspherical seamless capsules are made to maintain a good profile after the drying process. Thus, it is possible to obtain nonspherical seamless capsules showing a desired profile.

### Brief Description of the Drawings

[FIG. 1] A schematic illustration of an embodiment of seamless capsule manufacturing device according to the present invention, showing the configuration thereof.
[FIG. 2] Schematic illustrations of exemplar cross sections of the deformation section.
[FIG. 3] A schematic illustration of the profile of a seamless capsule that can be obtained when the cross section of FIG. 2(d) is used.
[FIG. 4] Schematic illustrations of the seamless capsules of Examples 3 through 6 and Reference Example 1.

### Explanation of Reference Symbols

- 1:: core liquid
- 2:: core liquid tank
- 3:: film forming liquid
- 4:: film forming liquid tank
- 5:: pump
- 6:: tube passage
- 7:: multiple nozzle
- 8:: pump
- 9:: tube passage
- 10:: hardening liquid
- 11:: flow passage tube
- 11A:: inflow section
- 11B:: outflow section
- 11C:: engaging section
- 12:: separator
- 13:: mesh
- 16:: separation tank
- 19:: pump
- 20:: tube passage
- 21:: cooling tank
- 22:: cooler
- 23:: pump
- 24:: tube passage
- 25:: inlet part
- 26:: liquid drop
- 27:: liquid drop
- 28:: deformation section
- 28a:: lead in part
- 28b:: formation tube part
- 28c:: lead out part
- D₀:: liquid drop diameter
- D₁:: formation tube part inner diameter
- D₂:: inlet part inner diameter
- L₁:: formation tube part length
- L₂:: length from inlet part upper end to formation tube part inlet
- S:: cross section of formation tube part
- SC:: seamless capsule

### Best Mode for Carrying Out the Invention

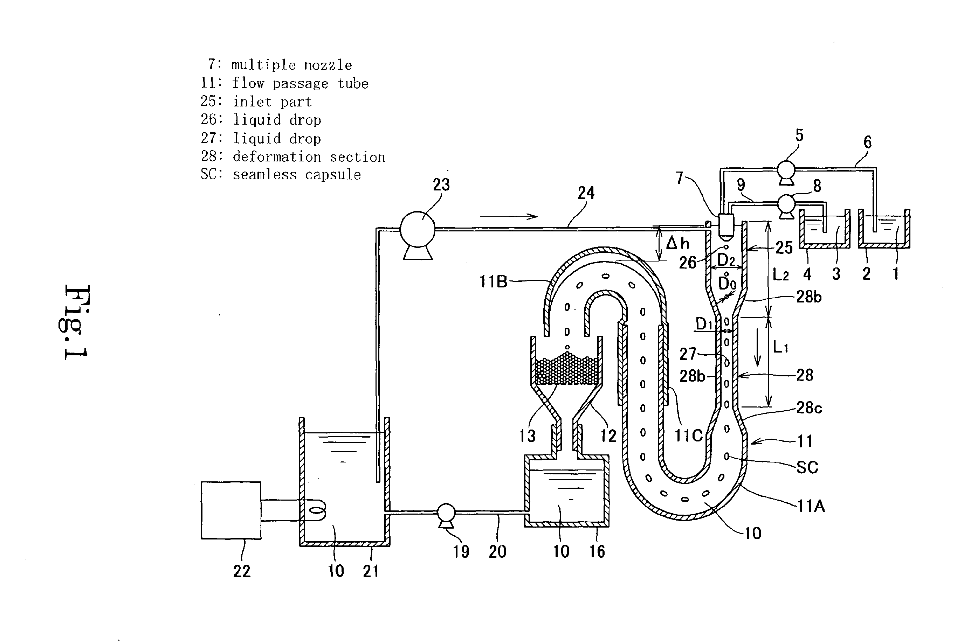

Embodiments of the present invention will be described below in detail with reference to the accompanying drawings. FIG. 1 is a schematic illustration of an embodiment of seamless capsule manufacturing device according to the present invention, showing the configuration thereof. Referring to FIG. 1, the seamless capsule manufacturing device is adapted to manufacture seamless capsules SC by ejecting liquid drops from a multiple nozzle 7 into a flow passage tube 11. Core liquid (inner layer liquid) 1 for forming seamless capsules is stored in a core liquid tank 2. Film forming liquid (outer layer liquid) 3 for coating each drop of core liquid 1 is stored in a film forming liquid tank 4. Core liquid 1 is fed by a pump 5 to the multiple nozzle 7 under pressure from the core liquid tank 2 by way of a tube passage 6. Film forming liquid 3 is fed to the multiple nozzle 7 under pressure from the film forming liquid tank 4 by way of a tube passage 9 and by means of a pump 8.

The flow passage tube 11 is provided at an upper inlet part thereof with an inflow section 11A of hardening liquid 10. Hardening liquid 10 is supplied to the inflow section 11A from the pump 23 by way of tube passage 24. The seamless capsule manufacturing device is of a so-called intra-liquid nozzle type and the multiple nozzle 7 is put into and placed in the inlet part 25 of the inflow section 11A. Core liquid 1 and film forming liquid 3 are ejected as capsule forming liquid from the multiple nozzle 7. The multiple nozzle 7 is subjected to vibrations applied to it by a vibration means (not shown) so that of the ejected liquid is separated by the vibrations to form a multilayer liquid drop 26 where a drop of core liquid 1 is coated by film forming liquid 3 all around its surface (to be referred to simply as liquid drop 26 hereinafter). In this way, liquid drops 26 are cooled and hardened as they are moved through hardening liquid 10 to produce seamless capsules SC. The temperature of hardening liquid 10 may be selected appropriately by considering the composition, the thickness and the temperature of the film forming liquid 3 as well as the size and the production rate of liquid drops 26. When the solid component of film forming liquid 3 contains gelatin as principal ingredient, the temperature of hardening liquid 10 is preferably between 4 and 16°C, more preferably between 5 and 10°C.

The volume of each liquid drop 2 6 can be determined by dividing the flown volume of the capsule forming liquid of the sum of the volume of the core liquid 1 and that of the film forming liquid 3 ejected from the multiple nozzle 7 per unit time by the number of liquid drops produced per unit time, that is, the number of vibrations per unit time of the multiple nozzle 7. The diameter D₀ of the ejected liquid drops in the inlet part is calculated from the volume of each liquid drop, assuming that the form of the liquid drop is a spherical shape.

The flow passage tube 11 is formed as a curved hollow cylinder having a substantially J-shaped inflow section 11A and an inverted J-shaped outflow section 11B that is telescopically linked to the inflow section 11A. The inflow section 11A and the outflow section 11B are engaged with and rigidly secured to each other at an engaging section 11C to provide a hermetically sealed condition. Alternatively, the inflow section 11A and the outflow section 11B may be linked to each other at the engaging section 11C so as to be vertically movable relative to each other. With such an arrangement, the difference Δh of the liquid level of the inflow section 11A and that of the outflow section 11B can be made variable and then it is possible to adjust the flow rate of hardening liquid 10 in the flow passage tube 11.

The inflow section 11A is provided at the top end thereof with an cylindrical inlet part 25 that is exposed to the multiple nozzle 7 and at the downstream side of the inlet part 25 with a deformation section 28. The deformation section 28 includes a lead in part 28a, a formation tube part 28b and a lead out part 28c. The lead in part 28a is directly connected to the lower end of the inlet part 25 and made to show a tapered profile with a gradually changing inner diameter. The formation tube part 28b is made to show a diameter smaller than the inlet part 25. More specifically, the inner diameter D₁ of the formation tube part 28b is smaller than the inner diameter D₂ of the inlet part 25 (D₁ < D₂). The lead out section 28c is directly connected to the lower end of the formation tube part 28b and made to show a tapered profile with a gradually changing inner diameter. The flow passage tube 11 arranged at the downstream side of the lead out part 28c is made to show a diameter same as the inlet part 25.

The deformation section 28 is arranged at a position where the liquid drops 26 ejected from the multiple nozzle 7 and passed through the inlet part 25 are still in a sol state. Preferably, the inner diameter D₁ of the formation tube part 28b is greater than and not greater than three times of the diameter D₀ of the ejected liquid drops 26 in the inlet part 25 (D₀ < D₁ ≤ 3D₀). In other words, if the flow passage tube 11 has a circular cross section, the cross sectional area S of the formation tube part 28b is defined to be within a range of (π/4)D₀2 < S ≤ (9π/4)D₀².

As the inner diameter D₁ and the cross sectional area S of the formation tube part 28b are defined in this way, a gap is formed between the liquid drops 26 and the inner surface of the formation tube part 28b to allow the liquid drops 26 to flow through the formation tube part 28b smoothly. Thus, it is possible to prevent the liquid drops 26 from being blocked in the deformation section 28 and the pulsation and the blocked condition, if any, of hardening liquid is suppressed. If the inner diameter D₁ and the cross sectional area S of the formation tube part 28b are defined to be excessively large, they provide little difference from those of the inlet part 25 and the change in the flow rate of hardening liquid is minimized to by turn minimize the effect of deforming the liquid drops 26. According to an experiment conducted by the inventors of the present invention, it was confirmed that an appropriate change is realized in the flow rate of hardening liquid and a satisfactory deformation effect is achieved when the inner diameter D₁ and the cross sectional area S of the formation tube part 28b are defined as above.

Additionally, the inner diameter D₁ of the formation tube part 28b is preferably between (1/6) times and (2/3) times of the inner diameter D₂, more preferably between (1/5) times and (3/5) times, most preferably between (1/4) times and (1/2) times of the inlet part. Similarly, the cross sectional area S of the formation tube part 28b is preferably between (1/36) times and (4/9) times, more preferably between (1/25) times and (4/25) times, most preferably between (1/16) times and (1/4) times of the cross sectional area of the inlet part 25. When the inner diameter D₁ and the cross sectional area S of the formation tube part 28b are defined in this way, it is possible to produce an appropriate change in the flow rate for turning the spherical liquid drops 26 into nonspherical liquid drops and obtain appropriately deformed nonspherical seamless capsules highly efficiently without giving rise to any ruptured liquid drops.

On the other hand, if the length from the upper end of the inlet part 25 to the inlet of the formation tube part 28b is L2, the length L1 of the formation tube part 28b is defined as L1 = 0.2 - 1.5 x L2. If the length L1 of the formation tube part 28b is too large, it is not possible to realize a flow rate of hardening liquid 10 necessary for forming capsules and the produced capsules block the passage of hardening liquid at the bottom part of the flow passage tube 11. When the length L1 of the formation tube part 28b is within the above range, it is possible to realize a flow rate of hardening liquid 10 flowing through the flow passage tube 11 necessary for forming capsules and provide a satisfactory deformation effect and a satisfactory production capacity.

A funnel-shaped separator 12 is arranged below the outlet end of the flow out section 11B. A mesh 13 is arranged in the separator 12 so as not to allow seamless capsules SC to pass but allow only hardening liquid 10 to pass through it. Thus, the seamless capsules SC and the hardening liquid 10 that flow out from the flow passage tube 11 together are separated by the separator 12 from each other. The hardening liquid 10 separated from the seamless capsules SC by the separator 12 is collected in the separation tank 16 arranged below. The hardening liquid 10 in the separation tank 16 is fed to a cooling tank 21 under pressure by way of a tube passage 20 and by means of a pump 19. Then, the hardening liquid 10 is cooled in the cooling tank 21 to a predetermined temperature level by means of a cooler 22. The hardening liquid 10 in the cooling tank 21 is then returned to the flow passage tube 11 by means of a pump 23.

A seamless capsule manufacturing device having the above-described configuration manufactures nonspherical seamless capsules in a manner as described below. Firstly, core liquid 1 and film forming liquid 3 are ejected from the multiple nozzle 7 to form spherical liquid drops 26 in the hardening liquid 10 in the flow passage tube 11. The liquid drops 26 then get to the deformation section 28 while they are still in a sol state. The flow rate of hardening liquid 10 rapidly rises in the deformation section 28 due to the change in the cross sectional area. Thus, the liquid drops 26 that are in a sol state are rapidly drawn into the formation tube part 28b of the deformation section 28 due to the change of flow rate. As the liquid drops 26 are rapidly drawn, the liquid drops 26 are expanded in the flowing direction of hardening liquid and flow out from the deformation section 28 as ellipsoidal or oblong liquid drops 27.

At this time, since the inner diameter D₁ of the formation tube part 28b is larger than the diameter D₀ of the liquid drops 26 ejected from the multiple nozzle 7, the inside of the formation tube part 28b is hardly clogged by liquid drops. In other words, the diameter of liquid drops is mechanically reduced with any of the conventional methods of manufacturing deformed soft seamless capsules that use a dropping method so that the diameter of the narrow tube and that of the mold are made smaller than the diameter of liquid drops as a matter of course and hence tubes or the like can be easily clogged by liquid drops. To the contrary, with a seamless capsule manufacturing method according to the invention that is adapted to be used with a seamless capsule manufacturing device according to the invention, the diameter of liquid drops 26 is changed by a change in the flow rate of hardening liquid so that the deformation section 28 is only required to show a change in the cross sectional area and it is possible to secure an inner diameter that is sufficiently large for allowing liquid drops 26 to pass through. Thus, the operation of the device is prevented from being suspended due to clogging of the tube and/or the mold to make it possible to raise the productivity. Additionally, the flow of hardening liquid is prevented from being pulsated and/or blocked by the resistance of liquid drops that arises when the latter are deformed. Then, the quality of finished capsules is made stable because dispersion of weight and uneven wall thickness of capsules are suppressed.

The liquid drops 27 that are deformed to become nonspherical in this way are then cooled in the deformation section 28 to become nonspherical seamless capsules SC. Subsequently, the seamless capsules SC flows down with hardening liquid 10 from the outlet of the outflow section 11B onto the mesh 13 of the separator 12. Then, the seamless capsules SC are separated from hardening liquid 10 by the mesh 13 and, when they gets to an appropriate amount, collected in a product collecting container (not shown) as batches of seamless capsules. On the other hand, the hardening liquid 10 are made to pass through the mesh 13 and collected in a separation tank 16.

Meanwhile, the moisture content ratio of the coat film of the seamless capsules SC (liquid drops 27), which are manufactured in this way, immediately after the separation from the hardening liquid 10 in a raw capsule condition is about 50 to 85wt%, although it may vary depending on the composition of the film coats. Problems arise when the moisture content ratio of the coat film are so high. The problems include that the coat films will show only a short storage life (because they are deteriorated by moisture), that they can easily be deformed by hand and that they are apt to be molten by the body temperature of the person who holds them or by the hot ambient temperature in summer. Therefore, it is necessary to dry the seamless capsules SC (liquid drops 27) after being separated from the hardening liquid 10 in order to improve the storage stability and handling ability.

It is preferable that the moisture content ratio of the coat film of the seamless capsules SC after the drying step is about 10 to 15wt%. Since the coat film are hydrophilic because of their composition, it is difficult to dry them below this level and it is desirable that the moisture content ratio is within the above cited range from the viewpoint of keeping an appropriate degree of softness and a comfortable touch. A moisture content ratio exceeding the above range is not desirable from the viewpoint of long storage life (because of the tendency of deterioration) and adhesion (because seamless capsules can easily adhere to each other and to the container containing them).

Seamless capsules are normally dried by aeration drying. Techniques of aeration drying that can be used for drying seamless capsules include the use of a fluidized bed drier and the use of a rotary drum type aeration drier, of which the user of a rotary drum type aeration drier is preferable for the purpose of the present invention. Dry air showing a low humidity is used for aeration drying. The temperature of air to be used for aeration drying is preferably between 10 and 30 °C, more preferably between 15 and 25°C. Procurement of dry air will be costly and the drying efficiency will be low when the temperature of dry air is lower than 10°C. On the other hand, the coat film (surfaces) of seamless capsules can become molten and air bubbles can be produced in the core liquid during the drying process when the temperature of dry air is higher than 30°C.

Meanwhile, when the manufactured seamless capsules SC (liquid drops 27) are subjected to aeration drying after the separation from hardening liquid 10, the nonspherical seamless capsules SC tend to gradually regain the original profile and become spherical. This phenomenon may be caused for the reasons as listed below. Firstly, as the seamless capsules SC are separated from cold hardening liquid 10 and subjected to aeration drying, using air whose temperature is higher than that of hardening liquid 10, the gelling power of the coat film (particularly gelatin) falls and the nonspherical seamless capsules SC tend to regain the original spherical profile, which is a stable profile for them. Secondly, the coat film of the seamless capsules SC lose moisture as they are dried so that they are subjected to force trying to contract them. Thirdly, the seamless capsules SC that are deformed to become nonspherical are subjected to force trying to make them round and spherical by the rotary motion (rolling effect) in the drying process using a fluidized bed drier or a rotary drum type aeration drier.

Thus, with known seamless capsule manufacturing methods, it is difficult to obtain seamless capsules showing a desired nonspherical profile because the seamless capsules that are made nonspherical by a method and device of the above-described type can be turned back to spherical in the subsequent drying process. However, the inventors of the present invention thought that the profile change in the drying process can be suppressed when seamless capsule SC that are made nonspherical are subjected to a drying process after a dehydration process and found that the profiles of nonspherical seamless capsules can be maintained by bringing them into contact with ethanol type processing liquid before an aeration drying process.

Ethanol type processing liquid that can be used for the purpose of the present invention contains ethanol as principal ingredient by not lower than 50 wt%, preferably not lower than 80%, more preferably between 90 and 99 wt%. Lecithin may be added to ethanol type processing liquid in order to prevent seamless capsules from adhering to each other. The ratio to which lecithin is added to ethanol type processing liquid is preferably 0.01 to 0.5 wt%, more preferably 0.1 to 0.2 wt%. Lecithin may be dissolved in medium-chain triglyceride (to be referred to as MCT hereinafter) before being added to ethanol type processing liquid.

When the composition of the coat film contains glycerin, glycerin may be lost with moisture from the coat film to modify the composition. If such is the case, glycerin may appropriately be added to ethanol type processing liquid for the purpose of suppressing the possible modification of the composition of the coat film. The rate at which glycerin is added varies depending on the composition of the coat film so that it is desirable that glycerin is added at such a rate that the glycerin concentration in the coat film is substantially equilibrated with the glycerin concentration of ethanol type processing liquid.

The technique to be used for bringing the manufactured nonspherical seamless capsules SC into contact with ethanol type processing liquid is not subjected to any particular limitations. In other words, any technique may be used so long as the deformation of the seamless capsules is maintained to a certain degree after the drying process. Note, however, that it is desirable that the manufactured nonspherical seamless capsules SC is brought into contact with ethanol type processing liquid after being separated from hardening liquid and before being subjected to an aeration drying process.

Additionally, the seamless capsules SC may be put into a centrifuge to reduce the amount of hardening liquid adhering to the surfaces thereof before the process of being brought into contact with ethanol type processing liquid. With this arrangement, hardening liquid is largely prevented from being mixed with ethanol type processing liquid to improve the productivity of the contact process.

The duration of the contact process of bringing the seamless capsules SC into contact with ethanol type processing liquid is not subjected to any particular limitations so long as the deformation of the seamless capsules is maintained to a certain degree after the drying process. The duration of the contact process is preferably not shorter than 3 minutes, more preferably not shorter than 5 minutes, most preferably between 10 and 90 minutes. However, it is particularly preferable that the duration of the contact process is between 20 and 60 minutes and the seamless capsules are desirably agitated during the contact process.

The longer the duration of the contact process of the nonspherical seamless capsules SC and ethanol type processing liquid, the degree of deformation of the nonspherical seamless capsules SC before the drying process tends to be maintained after the drying process. The profile of the nonspherical seamless capsules SC becomes invariable after the drying process when the duration of the contact process exceeds a certain period of time. Thus, the duration of the contact process of the nonspherical seamless capsules SC and ethanol type processing liquid can be selected appropriately by taking the desired profile (the degree of deformation) of the dried seamless capsules into consideration. More specifically, no contact process may be executed or the contact process may be conducted only for a short period of time before the aeration drying process when round and egg-shaped nonspherical seamless capsules are needed, whereas the contact process may be conducted for a relatively long period of time before the aeration drying process when sharp nonspherical seamless capsules are needed.

Note, the seamless capsules may be put into a centrifuge after the contact process of being brought into contact with ethanol type processing liquid to reduce the amount of ethanol type processing liquid to the surfaces thereof. With this arrangement, it is possible to raise the drying efficiency of the aeration drying process.

Examples of deformed seamless capsules according to the present invention will be described below. Although the present invention is by no means limited by the examples.

### [Example 1]

A filling liquid (core liquid) of medium-chain triglyceride (to be referred to as MCT hereinafter) as shown in Table 1 below was prepared. At the same time, a film forming liquid was prepared by heating and dissolving a mixture of the ingredients listed in Table 2 below at a mixing ratio also listed in Table 2. Then, conical deformed capsules were manufactured by means of a seamless capsule manufacturing device as illustrated in FIG. 1. The values of the parameters as listed in Table 3 below were used for the manufacture of the seamless capsules. The coat film ratio in Table 3 refers to the ratio of the mass of the coat film of a seamless capsule relative to the total mass of the seamless capsule.'

[Table 1]

**Table 1. Core liquid composition**

| | |
|---|---|
| MCT | 100 |
| total | 100 |

| | |
|---|---|
| (wt%) | |

[Table 2]

**Table 2. Film forming liquid composition**

| | |
|---|---|
| gelatin | 27 |
| glycerin | 3 |
| water | 70 |
| total | 100 |

| | |
|---|---|
| (wt%) | |

[Table 3]

**Table 3. Encapsulation parameters**

| | | Example 1 | Example 2 | Example 3 |
|---|---|---|---|---|
| liquid drop diameter during manufacture D₀ (mm) | | 5.7 | 7.9 | 5.7 |
| number of produced capsules (per second) | | 6 | 3 | 15 |
| cross section of formation tube D₁ of deformation section | | circular | elliptic | circular |
| formation tube diameter (mm) | D₂ | 22 | 22 | 22 |
| | D₁ | 8 | 10 | 22 |
| formation tube length (mm) | L₂ | 440 | 440 | 800 |
| | L₁ | 360 | 290 | - |
| hardening liquid | | MCT | MCT | MCT |
| hardening liquid flow rate (cm/sec) | D₂ | 5.8 | 4.6 | 12.2 |
| | D₁ | 41.3 | 22.4 | - |
| hardening liquid temperature (°C) | | 8 | 5 | 8 |
| capsule profile after drying bottom surface diameter height | | conical about 4. 9 mm about 5. 6 mm | echinus-shaped about 6.8 mm about 6 mm | spherical (diameter) 5 mm |
| coat film ratio after drying (%) | | 20 | 15 | 20 |

### [Example 2]

Core liquid and film forming liquid were prepared with the mixing ratios listed respectively in Tables 1 and 2 as in Example 1 and deformed capsules showing an echinus-shaped profile were manufactured by means of a seamless capsule manufacturing device as illustrated in FIG. 1. The values of the parameters as listed in Table 3 below were used for the manufacture of the seamless capsules.

### [Comparative Example 1]

In Comparative Example 1, core liquid and film forming liquid were prepared with the mixing ratios listed respectively in Tables 1 and 2 and spherical seamless capsules were manufactured by means of a seamless capsule manufacturing device that is similar to the one illustrated in FIG. 1 but does not have a deformation section 28b. The values of the parameters as listed in Table 3 above were used for the manufacture of the seamless capsules.

### [Example 3]

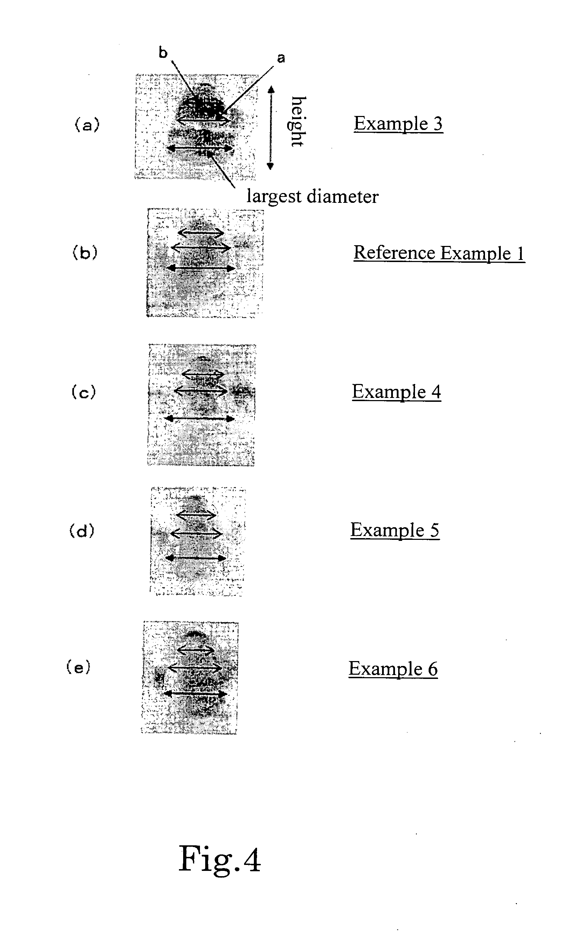

Conical deformed capsules were manufactured from a filling liquid (core liquid) as shown in Table 1 and a film forming liquid prepared by heating and dissolving a mixture of the ingredients listed in Table 2 at a mixing ratio also listed in Table 2 as in Example 1. The obtained deformed capsules were separated from hardening liquid to obtain conical deformed capsules (undried deformed capsules) . The values of the parameters as listed in Table 5 below were used for the manufacture of the seamless capsules. The profile of the deformed capsules is shown in FIG. 4(a).

The largest diameter in cross sections perpendicularly intersecting the vertical direction of the conical seamless capsule and the position (height) thereof were determined, provided that the major axis of the conical seamless capsule is expressed as height (h). It may be safe to assume that the profile of the seamless capsule comes close to spherical from conical (nonspherical) as the ratio of the largest diameter relative to h comes close to 1 and the position (height) of the largest diameter comes close to the center. The ratio of the largest diameter relative to the height of the conical seamless capsule obtained in Example 3 before an aeration drying process was as small as 0.77 to prove that the seamless capsule was nonspherical.

Additionally, the diameter of the cross sectional circle at the height of the middle point of the height of the largest diameter from the bottom surface of the conical seamless capsule and the height of the apex of the conical seamless capsule was defined as a. The diameter of the cross sectional circuit at the height of the middle point of the height of a and the height of the apex was defined as b. It may be safe to assume that the profile of the seamless capsule is close to conical when the value of the diameters a and b are sequentially and largely reduced from the largest diameter. The values of the diameters a and b relative to the height of the conical seamless capsules obtained in Example 3 before the aeration drying process were smaller than the value of the largest diameter to evidence the characteristics of a cone.

### [Reference Example 1]

The deformed seamless capsules manufactured in Example 3 were subjected to an aeration drying process (using a rotary drum type aeration drier with drying air temperature of 20°C) to obtain dried and deformed seamless capsules. The values of the parameters as listed in Table 5 below were used for the manufacture of the seamless capsules. The profile of the deformed capsules is shown in FIG. 4(b).

The deformed seamless capsules after the aeration drying process showed a reduced height (h) for the apex if compared with the deformed seamless capsules before the drying process. Additionally, the largest diameter was increased and the position (height) of the largest diameter was raised after the aeration drying process. Thus, by comparing the deformed seamless capsules before the drying process and those after the drying process, it was clearly recognized that the profile was rounded and changed from conical to egg-shaped. Additionally, the values of the diameters a and b were increased after the drying process if compared with the values before the drying process and the ratio relative to the height (h) were also clearly increased.

### [Example 4]

100g of the deformed seamless capsules before an aeration drying process as manufactured in Example 3 were subjected to a contact process where they are brought into contact with 150g of ethanol type processing liquid prepared to show a composition ratio as shown in Table 4 and stirred for 10 minutes and subsequently to an aeration drying process (using a rotary drum type aeration drier with drying air temperature of 20°C) to obtain dried and deformed seamless capsules. The values of the parameters as listed in Table 5 below were used for the contact process using ethanol type processing liquid (to be referred to as ethanol process hereinafter) and the manufacture of the seamless capsules. The profile of the deformed capsules is shown in FIG. 4(c).

The deformed seamless capsules that were subjected to an ethanol process and subsequently to an aeration drying process showed a slightly reduced height (h) for the apex but the largest diameter thereof remained substantially same. While they were rounded slightly after the drying process if compared with the seamless capsules before the drying process, they retained the profile of conical deformed seamless capsule. Additionally, the values of the diameters a and b were not changed significantly.

### [Example 5]

Conical deformed seamless capsules were prepared as in Example 4 except that the deformed seamless capsules manufactured as in Example 3 were subjected to a contact process where they were brought into contact with ethanol type processing liquid and stirred for 20 minutes. The values of the parameters as listed in Table 5 below were used for the ethanol process and the manufacture of the seamless capsules. The profile of the deformed capsules is shown in FIG. 4(d).

The deformed seamless capsules subjected to an ethanol process for 20 minutes before an aeration drying process did not show any significant change in the height (h) of the apex and the largest diameter. The values of the diameters a and b and the ratio relative to the height (h) before the drying process did not change significantly. Thus, they retained the conical profile before the drying process until after the aeration drying process.

### [Example 6]

Conical deformed seamless capsules were prepared as in Example 4 except that the deformed seamless capsules manufactured as in Example 3 were subjected to a contact process where they were brought into contact with ethanol type processing liquid and stirred for 60 minutes. The values of the parameters as listed in Table 5 below were used for the ethanol process and the manufacture of the seamless capsules. The profile of the deformed capsules is shown in FIG. 4(e).

The deformed seamless capsules subjected to an ethanol process for 60 minutes before an aeration drying process did not show any significant change in the height (h) of the apex and the largest diameter. The values of the diameters a and b and the ratio relative to the height (h) did not change significantly. Thus, they retained the conical profile before the drying process until after the aeration drying process.

The profile of the deformed seamless capsules subjected to an ethanol process for 60 minutes (Example 6) was not significantly different from and substantially same that of the deformed seamless capsules subjected to an ethanol process for 20 minutes (Example 5) when observed after the aeration drying process.

It may be understood that the degree of deformation (sharpness) of deformed (nonspherical) seamless capsules is tend to be maintained when the duration of the contact process using ethanol type processing liquid is longer and that the profile of deformed (nonspherical) seamless capsules is held unchanged after a drying process when the duration of the contact process exceeds a certain time period (20 minutes in the case of this embodiment) .

Thus, the profile of the seamless capsules that are deformed to become nonspherical is held unchanged through a drying process when they are subjected to a contact process using an ethanol type processing liquid before the drying process so that it is possible to maintain the degree of deformation of the nonspherical seamless capsules remarkably well after the drying process. Therefore, while it is difficult to obtain nonspherical seamless capsules showing a desired profile by any known method, it is possible to obtain nonspherical seamless capsules showing a desired profile with ease by a method according to the present invention.

[Table 4]

**Table 4. Ethanol processing liquid composition**

| | |
|---|---|
| ethanol | 98.4 |
| MCT | 1.5 |
| lecithin | 0.1 |
| total | 100 |

| | |
|---|---|
| (wt%) | |

[Table 5]

**Table 5. Encapsulation parameters and duration of ethanol process**

| | | example 4 | example 5 | example 6 | ref. ex. 1 | example 3 |
|---|---|---|---|---|---|---|
| particle diameter (mm) | | 7 | 7 | 7 | 7 | 7 |
| coat film ratio after drying (%) | | 20 | 20 | 20 | 20 | 20 |
| liquid drop diameter during manufacture D₀ (mm) | | 7.9 | 7.9 | 7.9 | 7.9 | 7.9 |
| number of produced capsules (per second) | | 3 | 3 | 3 | 3 | 3 |
| cross section of formation tube D₁ of deformation section | | circular | circular | circular | circular | circular |
| formation tube diameter (mm) | D₂ | 22 | 22 | 22 | 22 | 22 |
| | D₁ | 10 | 10 | 10 | 10 | 10 |
| formation tube length (mm) | L₂ | 440 | 440 | 440 | 440 | 440 |
| | L₁ | 290 | 290 | 290 | 290 | 290 |
| hardening liquid | | MCT | MCT | MCT | MCT | MCT |
| hardening liquid flow rate (cm/sec) | D₂ | 4.6 | 4.6 | 4.6 | 4.6 | 4.6 |
| | D₁ | 22.4 | 22.4 | 22.4 | 22.4 | 22.4 |
| hardening liquid temp. (°C) | | 5 | 5 | 5 | 5 | 5 |
| use or nonuse of drying process | | used | used | used | used | not used |
| capsule profile after drying | | conical | conical | conical | egg-shaped | (conical) |
| duration of ethanol process (min) | | 10 | 20 | 60 | 0 | 0 |
| capsule dimensions (mm) Before drying in Example 3, after drying in other examples | height (mm) | 8.5 | 9.5 | 9.5 | 8.0 | 9.3 |
| | largest dia. (mm) | 7.3 (0.86) | 7.0 (0.74) | 7.0 (0.74) | 7.5 (0.94) | 7.2 (0.77) |
| | diameter a (mm) | 6.0 (0.71) | 5.8 (0.61) | 5.8 (0.61) | 6.4 (0.8) | 6.1 (0. 66) |
| | diameter b (mm) | 4.0 (0.47) | 4.4 (0.46) | 4.5 (0.47) | 4.8 (0.6) | 4.4 (0.47) |

| | | | | | | |
|---|---|---|---|---|---|---|
| #1: The value in ( ) indicates the ratio relative to height. | | | | | | |
| #2: The diameter a indicates the diameter (mm) at the middle point | | | | | | |
| of the height of the largest diameter and the height of the apex. | | | | | | |
| #3: The diameter b indicates the diameter (mm) at the middle point of the height of the diameter a and the height of the apex. | | | | | | |

The present invention is not limited to the above embodiments and various changes and modifications can be made within the spirit and scope of the present invention.

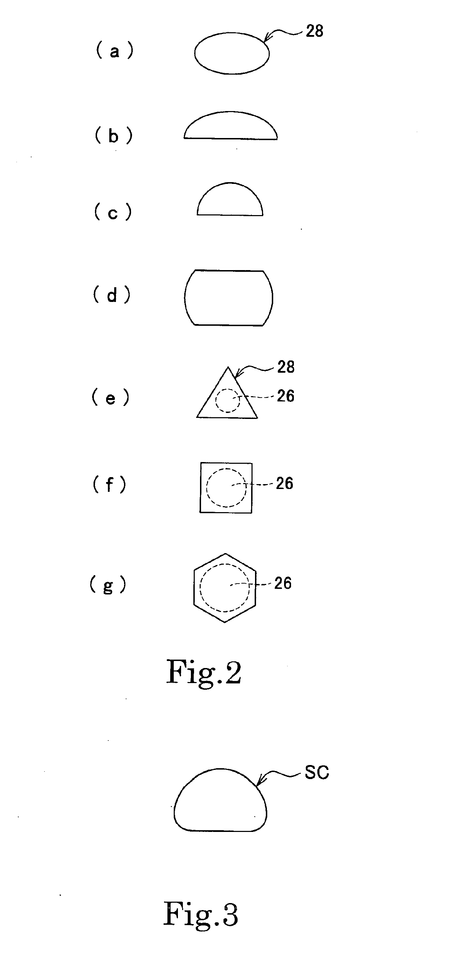

The contour of the cross section of the formation tube part 28b is not limited to circular and may alternatively be that of an ellipse (FIG. 2(a)), a shape having one or more than one straight lines (FIGS. 2(b) through 2(d)) or that of a polygon formed only by using straight lines (FIGS. 2(e) through 2(g)). In any cross section, the size of the smallest part is defined to be larger than the liquid drop diameter so that liquid drops 26 may smoothly pass through (see FIGS. 2(e) through 2(g)).

When the formation tube part 28b is made to show a cross section as illustrated in FIG. 2(d), the produced seamless capsules SC will show the profile of a semi-ellipsoid (echinus-shaped) having a flat surface part as shown in FIG. 3. Such echinus-shaped capsules can stably "sit" if compared with ellipsoidal capsules. Hence, according to the present invention, it is possible to manufacture capsules that would not roll and hence are stable with ease.

A multiple nozzle 7 is not limited to a double nozzle as described above and may alternatively be a triple nozzle. Vibrations techniques that can be used to produce multilayer liquid drops include a technique of applying vibrations to a ring or a tube in addition to the above-described technique of applying vibrations to a nozzle. The nozzle for ejecting liquid drops may be a single nozzle adapted to eject liquid drops of a single layer.

Additionally, the composition and the ingredients of the inner layer and those of the outer layer of multilayer liquid drops for forming seamless capsules may be selected appropriately. The flow passage tube is not limited to an S-shaped tube but may alternatively be a downwardly extending spiral tube. For the purpose of the present invention, liquid drops may be hardened by way of a chemical reaction instead of by cooling.

The use of an ethanol process as described above is not limited to seamless capsules produced by a manufacturing device as illustrated in FIG. 1 and a manufacturing method to be used with the manufacturing device. In other words, an ethanol process may also be used for seamless capsules produced by some other manufacturing device and a manufacturing method to be used with such a manufacturing device. For example, seamless capsules manufactured by means of a known manufacturing method such as the one described in Japanese Patent Publication No. 60-46980 may also be subj ected to an ethanol process so as to maintain the nonspherical profile thereof after a drying process.

## Claims

1. A seamless capsule manufacturing method of manufacturing seamless capsules by ejecting liquid drops from a nozzle into hardening liquid and hardening at least a surface part of each liquid drop, **characterized in that**
each liquid drop is deformed to show a nonspherical profile by changing the flow rate of hardening liquid while the liquid drop is still in a sol state.

2. The method according to claim 1, **characterized in that** the liquid drop is drawn to expand in the direction of the flow path by increasing the flow rate of hardening liquid.

3. The method according to claim 1 or 2, **characterized in that** the formed nonspherical seamless capsules are subjected to a contact process of being brought into contact with ethanol type processing liquid.

4. A seamless capsule manufacturing device comprising a nozzle for ejecting liquid for forming capsules and a flow passage tube containing hardening liquid for hardening at least a surface part of each liquid drop formed from the liquid, **characterized in that**
the flow passage tube has an inlet part exposed to the nozzle so as to receive the liquid ejected/supplied from the nozzle and a deformation section having a cross sectional area smaller than the inlet part.

5. The device according to claim 4, **characterized in that**
the deformation section is arranged downstream relative to the inlet part at a position where the ejected liquid drops arrive in a sol state.

6. The device according to claim 4 or 5, **characterized in that**
the cross section of the deformation section is elliptic.

7. The device according to claim 4 or 5, **characterized in that**
the cross section of the deformation section shows a contour having one or more than one straight lines.

8. The device according to claim 4 or 5, **characterized in that**
the cross section of the deformation section is polygonal.

9. The device according to claim 4, **characterized in that**,
if the diameter of the largest circle that can be inscribed in the inner periphery of the deformation section is D₁, the D₁ is greater than the diameter D₀ of the ejected liquid drops and not greater than three times of the diameter D₀ of the ejected liquid drops in the inlet part (Do < D₁ ≤ 3D₀) .

10. The device according to claim 4, **characterized in that**,
if the diameter of the largest circle that can be inscribed in the inner periphery of the deformation section is D₁, the D₁ is between one sixth times and two third times of the inner diameter D₂ of the flow passage tube in the inlet part.

11. The device according to claim 4, **characterized in that**
if the diameter of the ejected liquid drops in the inlet part is D₀, the cross sectional area S of the deformation section is defined to be within a range of (π/4)D₀² < S ≤ ( 9π/4) D₀².

12. The device according to claim 4, **characterized in that**
the cross sectional area S of the deformation section is between one thirty sixth times and four ninth times of the cross sectional area of the flow passage tube in the inlet part.

13. A seamless capsule manufacturing method,
**characterized by** manufacturing nonspherical seamless capsules by means of a seamless capsule manufacturing device according to claim 4.

14. The seamless capsule manufacturing method according to claim 13, **characterized in that** a contact process of brining the seamless capsules into contact with ethanol type processing liquid is additionally conducted on the seamless capsules.

15. A nonspherical seamless capsule obtained by means of a seamless capsule manufacturing method according to claim 13 or 14.
